# FASCICULE DE BREVET EUROPEEN

(11) **EP 2 926 863 B1**
(45) Date de publication et mention de la délivrance du brevet: **31.07.2019**
(21) Numéro de dépôt: 15157914.1
(22) Date de dépôt: 06.03.2015
(51) Int. Cl.: A61N 1/372, A61N 1/36, A61N 1/368

(54) **Utilisation d'un générateur cardiaque implantable de stimulation, défibrillation et/ou resynchronisation du myocarde comme générateur VNS de stimulation du nerf vague**
Verwendung eines implantierbaren Herzschrittmachers zur Stimulation, Defibrillation und/oder Resynchronisation des Herzmuskels als Impulsgenerator zur Vagus-Nerv-Stimulation (VNS)
Use of an implantable cardiac generator for myocardial stimulation, defibrillation and/or resynchronisation as a vagus nerve stimulation (VNS) generator

(30) Priorité: 01.04.2014 FR 1452863
(43) Date de publication de la demande: 07.10.2015
(73) Titulaire: Sorin CRM SAS, 92140 Clamart (FR)
(72) Inventeur: Legay, Thierry, 91640 Fontenay les Briis (FR); Blumstein, Hervé, 28130 Mévoisins (FR); Chatalic, Guillaume, 92140 Clamart (FR); Henry, Christine, 75014 Paris (FR)
(74) Mandataire: Grünecker Patent- und Rechtsanwälte PartG mbB

(56) Documents cités:
- US-A1- 2006 079 942
- US-A1- 2006 217 772
- US-A1- 2011 009 914
- US-A1- 2011 276 103
- US-A1- 2012 185 010
- US-A1- 2012 290 030

## Description

L'invention concerne les "dispositifs médicaux implantables actifs" tels que définis par la directive 90/385/CEE du 20 juin 1990 du Conseil des communautés européennes.

Elle concerne plus précisément les implants permettant de délivrer des thérapies de stimulation du système nerveux, notamment de stimulation du nerf vague. Ce type de stimulation sera ici désigné "neurostimulation" ou "stimulation VNS" (*Vagus Nerve Stimulation*).

La stimulation du système nerveux est une thérapie reconnue à l'égard de très nombreux troubles tels que l'épilepsie, la douleur, l'insuffisance cardiaque, l'apnée, l'obésité, etc. Pour le traitement des troubles tels que l'insuffisance cardiaque, l'épilepsie ou l'obésité, les dispositifs utilisés comprennent typiquement une sonde pourvue d'une électrode implantée sur le nerf vague (dite "sonde VNS") et un générateur délivrant des impulsions VNS sur cette électrode.

Dans certaines thérapies, le nerf vague est stimulé de façon synchrone avec le rythme cardiaque, le dispositif comprenant alors des moyens de recueil des ondes de dépolarisation du myocarde, typiquement des moyens de recueil d'un ECG par une électrode subcutanée, ou d'un EGM par une électrode implantée sur ou dans le myocarde. La stimulation VNS consiste alors à délivrer un "profil de stimulation" comprenant des salves de quelques impulsions VNS déclenchées en synchronisme avec le rythme cardiaque. Ce type de stimulation VNS est en particulier bien adapté au traitement des troubles cardiaques, notamment chez les patients en risque d'insuffisance cardiaque, où la stimulation du nerf vague agit sur les fonctions cardiovasculaires par réduction du rythme cardiaque, réduction de la contractilité du myocarde et augmentation de la durée de la diastole, ce qui peut aider à réduire l'évolution d'un remodelage cardiaque susceptible de mener à un état d'insuffisance cardiaque aggravée.

Dans d'autres types de thérapie, le profil de stimulation VNS est composé de salves ou trains d'impulsions répétitives à haute fréquence (plusieurs dizaines de hertz) produites pendant des périodes dites "d'activité" de quelques dizaines de secondes entrecoupées par des périodes "d'inactivité" de quelques minutes durant lesquelles la stimulation n'est plus délivrée.

De façon générale, dans le cas des pathologies cardiaques, notamment de l'insuffisance cardiaque, l'implantation d'un dispositif cardiaque est fréquemment indiquée, qu'il s'agisse d'un stimulateur (PM, *Pacemaker*), d'un défibrillateur implantable (ICD, *Implantable Cardioverter*/*Defibrillator*) ou d'un resynchronisateur (dispositif CRT, *Cardiac Resynchronization Therapy*). Cette stimulation, opérant par délivrance d'impulsions au myocarde par exemple au moyen d'une sonde endocavitaire, sera désignée par la suite "stimulation cardiaque" ou "stimulation CRM" (*Cardiac Rhythm Management*).

Les patients déjà porteurs d'un tel dispositif cardiaque implanté, ou susceptibles de le recevoir, peuvent également tirer bénéfice d'une stimulation VNS.

Pour pouvoir délivrer indifféremment une stimulation CRM ou une stimulation VNS à partir d'un même implant, le US 2006/0217772 A1 propose de réaliser un appareil comprenant un circuit permettant de générer sur un même canal soit des impulsions CRM soit des impulsions VNS. Le générateur d'impulsions est commun, mais il est piloté avec des paramètres d'amplitude d'impulsion (c'est-à-dire de tension délivrée aux électrodes) et de fréquence de récurrence des impulsions qui dépendent de la fonction sélectionnée, CRM ou VNS.

L'application au nerf vague d'impulsions générées par un tel appareil est toutefois susceptible de produire un effet physiologique très variable, car l'interface nerveuse électrode/nerf vague est beaucoup plus instable que l'interface cardiaque électrode/myocarde : on constate en effet des changements importants de l'impédance biologique au niveau de cette interface ce qui, pour une même tension de stimulation, conduit à des intensités de courant très variables circulant dans les zones du nerf vague à stimuler, avec pour conséquence des effets physiologiques pouvant varier de manière erratique en raison de ces intensités variables et imprévisibles. Concrètement, les enseignements de ce document ne permettent pas de réaliser un dispositif procurant une stimulation VNS satisfaisante, quand bien même le générateur de stimulation cardiaque serait programmé pour délivrer les impulsions à une cadence et selon un profil adaptés à une stimulation VNS.

Le US 2011/0276103 A1 décrit un autre appareil mixte, comprenant un circuit propre à générer sur un même canal soit des impulsions CRM soit des impulsions VNS. Le générateur d'impulsions comprend des circuits de charge et de décharge contrôlées d'un condensateur, avec une source de courant commutable montée en série dans le circuit de décharge. Dans le cas d'une stimulation CRM, le trajet de décharge contourne cette source de courant qui est neutralisée, de sorte que la tension de décharge du condensateur est directement appliquée aux électrodes reliées au générateur. En revanche, dans le cas d'une stimulation VNS la source de courant insérée dans le trajet de décharge du condensateur a pour effet de réguler le courant de l'impulsion délivrée, et permet donc d'ajuster de façon précise la quantité de charge appliquée au nerf en contact avec les électrodes.

Il est ainsi possible de combiner dans un même appareil les fonctionnalités de stimulation CRM et de stimulation VNS. Toutefois, le dispositif est spécifiquement conçu au départ pour assurer cette double fonction, et ceci au prix d'une complexité accrue (présence d'une source de courant, organes de commutation divers, etc.) donc d'un coût global élevé, tant au stade du développement qu'à celui de la fabrication.

Le besoin subsiste donc de disposer d'un générateur CRM/VNS mixte simplifié, notamment au niveau *hardware* (matériel), avec des circuits conventionnels basés sur la charge/décharge contrôlées d'un condensateur, mais ne mettant en oeuvre ni source de courant ni organes de commutation additionnels.

Ainsi, un premier but de l'invention est la réduction du coût unitaire des dispositifs destinés à la stimulation VNS, en reprenant pour cela une partie *hardware* identique à ce qui existe déjà dans un stimulateur cardiaque implantable connu. Une telle manière de procéder autoriserait une mise en oeuvre sans surcoût important, en partant d'un appareillage existant et éprouvé ne nécessitant que des adaptations mineures.

Un autre aspect de la présente invention résulte de la constatation que chez certains patients ayant reçu un resynchronisateur cardiaque (dispositif CRT) une proportion relativement importante, de l'ordre de 30 %, de ces patients ne répondent pas à la thérapie CRT et que l'implantation du dispositif n'a procuré aucun effet positif. En revanche, ces patients pourraient être répondeurs à une thérapie VNS, mais dans ce cas il serait nécessaire d'utiliser un autre dispositif, nécessitant de ce fait une nouvelle intervention pour explanter le dispositif CRT et implanter un dispositif VNS, avec tous les inconvénients et les coûts associés à une telle intervention.

Un autre but de l'invention est de pouvoir disposer d'un générateur VNS utilisable pour les catégories de patients décrits ci-dessus, notamment pour des patients déjà porteurs d'un stimulateur/défibrillateur cardiaque qui pourraient faire l'objet avec profit d'une stimulation VNS complémentaire. Pour ces patients, il serait bien préférable de disposer d'un dispositif, notamment d'un générateur, combinant en un seul boitier les deux thérapies (cardiaque et VNS), avec un avantage significatif aussi bien pour le confort du patient que pour le coût supporté par le système de santé.

L'idée de base de l'invention consiste à utiliser un générateur de stimulation cardiaque, c'est-à-dire originellement destiné à une stimulation d'une ou plusieurs cavités (ventricule ou oreillette) du myocarde, et à adapter ce dispositif pour le rendre capable d'une stimulation du nerf vague en lieu et place d'une stimulation d'un ventricule ou d'une oreillette.

Comme on le verra à la lecture de la description détaillée, cette adaptation pourra être réalisée par une modification simple du seul logiciel de pilotage du générateur cardiaque. Ainsi, la partie matérielle du dispositif de stimulation VNS étant identique à ce qui existe déjà pour un stimulateur cardiaque, la stimulation VNS pourra, grâce à l'invention, être mise en oeuvre sans surcoût important - notamment sans ajout d'une source de courant ni d'organes de commutation additionnels et en conservant l'architecture conventionnelle des circuits de charge/décharge contrôlés en tension.

En particulier, comme on le verra également, cette adaptation permettant de transformer un stimulateur cardiaque en stimulateur VNS pourra être effectuée même chez un patient déjà implanté, en conservant le même générateur - donc sans explantation de ce dispositif - et en connectant sur l'une des voies du générateur (par exemple la voie auriculaire) une sonde VNS en lieu et place de la sonde cardiaque. Une autre voie reliée à une sonde cardiaque (par exemple la voie ventriculaire reliée à une sonde endocavitaire) pourra être éventuellement utilisée pour la synchronisation des impulsions VNS sur le rythme cardiaque, si un tel profil de neurostimulation est choisi.

Ce principe est applicable non seulement à un simple stimulateur cardiaque (*pacemaker*) mais également à un défibrillateur implanté (donc en conservant la capacité de délivrer un choc de thérapie antitachycardique). Il est également applicable à un resynchronisateur (dispositif CRT), en réutilisant l'une des voies ventriculaires pour l'adapter aux fins d'une stimulation VNS, l'autre voie ventriculaire étant conservée pour le recueil d'un signal de synchronisation de la thérapie VNS et pouvant éventuellement continuer à servir à la délivrance d'impulsions de stimulation cardiaque conjointement à la stimulation VNS.

Ce même principe peut être extrapolé, pour les patients qui relèvent d'une telle indication, à la substitution d'une stimulation VNS à une stimulation cardiaque, toutes les voies du générateur de stimulation cardiaque (simple, double ou triple chambre) étant réutilisées comme autant de canaux de neurostimulation, par connexion à une ou plusieurs sondes respectives. On notera que, si le patient est déjà implanté, cette transformation peut être effectuée sans explantation du générateur, l'intervention ne portant que sur la mise en place d'une ou plusieurs sondes VNS et leur raccordement au générateur (avec ou sans explantation, le cas échéant, des sondes cardiaques devenues sans objet).

Cependant, dans tous ces cas de figure, un générateur de stimulation cardiaque ne convient pas à la délivrance d'impulsions VNS.

En effet, comme expliqué plus haut, l'application au nerf vague d'impulsions à tension constante produirait un effet physiologique imprévisible du fait à des intensités de courant très variables circulant dans les zones du nerf vague à stimuler, en produisant des effets physiologiques pouvant varier de manière erratique en raison de ces intensités variables et imprévisibles.

De ce fait, la simple substitution d'une sonde cardiaque par une sonde VNS ne permet pas d'atteindre le résultat souhaité, quand bien même le générateur de stimulation cardiaque serait programmé pour délivrer les impulsions à une cadence et selon un profil adaptés à une stimulation VNS.

Pour résoudre ce problème, l'invention propose d'adapter ce générateur de manière à contrôler dynamiquement la quantité de charge électrique unitaire délivrée au nerf vague par les impulsions générées, en ajustant cette quantité de charge à une valeur sensiblement constante d'une impulsion à la suivante et d'une salve à la suivante.

En particulier, l'invention propose d'utiliser le module de mesure de l'impédance qui est présent dans la plupart des dispositifs cardiaques pour évaluer la charge électrique délivrée au nerf vague par l'électrode de stimulation, et ajuster en conséquence la tension d'impulsion de manière à amener à une valeur cible constante la quantité de charge mesurée.

Essentiellement, un tel module évalue la chute de tension aux bornes du condensateur réservoir à partir duquel est délivrée l'impulsion de stimulation, entre le début et la fin de cette impulsion. L'analyse de cette chute de tension donne une estimation de la quantité de charge délivrée au nerf vague pendant la durée de l'impulsion, et cette mesure pourra servir de variable d'asservissement afin d'ajuster la tension de charge préalable du condensateur (tension en début d'impulsion) de manière à assurer la délivrance d'une quantité de charge impulsionnelle constante, d'une impulsion VNS à la suivante et d'une salve VNS à la suivante.

En d'autres termes, l'invention propose de "monitorer" en continu l'impédance de sortie et d'ajuster à chaque impulsion la tension de charge du condensateur pour réguler le courant de décharge, et donc la quantité de charge appliquée au nerf vague - et ceci sans aucun recours à une source de courant (source qui est d'ailleurs généralement absente des générateurs cardiaques conventionnels).

On soulignera le fait que, de façon particulièrement avantageuse, l'adaptation du générateur peut être réalisée par une simple modification du logiciel de pilotage du circuit de stimulation, sans aucun changement des différents organes matériels : tous les circuits étant déjà présents, il convient seulement de les faire fonctionner différemment, par implantation d'un module logiciel dédié dans le générateur - alors même qu'il est impossible de programmer une fonctionnalité de source de courant par des moyens purement logiciels.

Plus précisément, l'invention propose un dispositif comprenant, de manière en elle-même connue d'après le US 2006/0217772 A1 précité : un générateur cardiaque implantable de stimulation, défibrillation et/ou resynchronisation du myocarde comportant au moins deux bornes de détection/stimulation avec une première et une seconde borne ; un circuit de stimulation apte à générer des impulsions électriques de stimulation ; un circuit de pilotage apte à contrôler la délivrance au moins sur la première borne des impulsions générées par le circuit de stimulation et le séquencement de ces impulsions ; et un circuit de recueil d'un signal EGM d'électrogramme endocavitaire et de détection d'une onde de dépolarisation cardiaque spontanée ou stimulée sur chaque cycle cardiaque.

Le dispositif comprend en outre au moins une sonde de stimulation du nerf vague, VNS, apte à être implantée sur le nerf vague ou à proximité de celui-ci. La sonde VNS est pourvue de moyens de connexion adaptés à un générateur cardiaque, et elle est reliée à la première borne du générateur cardiaque de manière à recevoir les impulsions produites sur cette borne par le générateur cardiaque afin d'appliquer ces impulsions sur le nerf vague. Le circuit de pilotage du générateur cardiaque est apte à contrôler le circuit de stimulation de manière à produire des salves d'impulsions, ces salves étant des salves VNS générées continûment en succession pendant des périodes d'activité séparées par des périodes intercalaires d'inactivité, et/ou générées en synchronisme sur détection d'une onde de dépolarisation cardiaque.

De façon caractéristique de l'invention, le circuit de pilotage est apte à mesurer l'impédance de la sonde VNS et, en réponse, à contrôler dynamiquement la tension d'impulsion générée par le circuit de stimulation de manière à ajuster à une valeur constante, d'une impulsion à la suivante et d'une salve à la suivante, la charge délivrée par chaque impulsion de stimulation.

De préférence, les moyens de connexion adaptés à un générateur cardiaque comprennent un connecteur de type IS-1 monté sur la sonde VNS.

Dans une forme de réalisation préférentielle particulièrement avantageuse, le circuit de stimulation comprend un condensateur et des moyens de charge et de décharge du condensateur sélectivement contrôlés par le circuit de pilotage. Le circuit de pilotage, quant à lui, comprend : des moyens de mesure i) de la tension de charge préalable du condensateur et ii) de la chute de la tension aux bornes de ce condensateur pendant la durée de la décharge ; des moyens de calcul, à partir desdites mesures de tension de charge préalable et de chute de tension, de la charge électrique effectivement délivrée par l'impulsion de stimulation produite par la décharge du condensateur ; des moyens de calcul de l'écart entre la charge électrique ainsi calculée et une valeur de consigne correspondant à ladite valeur constante de charge électrique délivrée à chaque impulsion; et des moyens d'ajustement de la tension de charge préalable du condensateur en fonction de l'écart ainsi calculé, dans un sens allant dans la réduction de cet écart.

L'invention a également pour objet un logiciel de conversion d'un générateur cardiaque implantable en un générateur VNS.

L'utilisation d'un générateur cardiaque implantable tel que ci-dessus, en combinaison avec une sonde de stimulation VNS apte à être implantée sur le nerf vague ou à proximité de celui-ci, pour l'obtention d'un dispositif médical actif VNS et un procédé de conversion d'un générateur cardiaque implantable de stimulation, défibrillation et/ou resynchronisation du myocarde en un générateur VNS selon la technique précitée sont des exemples de réalisation qui ne font pas partie de la présente invention mais qui sont utiles pour sa compréhension.

On va maintenant décrire un exemple de mise en oeuvre de l'invention, en référence aux dessins annexés où les mêmes références désignent d'une figure à l'autre des éléments identiques ou fonctionnellement semblables.
La Figure 1 est une vue générale de présentation du dispositif médical de l'invention, montrant le générateur et le nerf vague ainsi que la sonde VNS utilisée.
La Figure 2 est un chronogramme explicitant un premier profil de neurostimulation, dans lequel les impulsions VNS sont délivrées sous forme de brefs trains d'impulsions synchronisés sur des événements cardiaques.
La Figure 3 est un chronogramme explicitant un second profil de neurostimulation, dans lequel les impulsions VNS sont délivrées sous forme de trains d'impulsions prolongés, délivrés de façon non synchrone au cours de périodes prédéterminées de stimulation alternant avec des intervalles de repos de durée également prédéterminée.
La Figure 4 est une représentation schématique par blocs des circuits de stimulation et de pilotage de l'un des canaux du générateur utilisé pour la mise en oeuvre de l'invention.
La Figure 5 illustre le profil d'une impulsion de stimulation délivrée par les circuits de la Figure 4, avec les différents paramètres impliqués dans la mise en oeuvre de l'invention pour que cette impulsion puisse être appliquée au nerf vague en tant qu'impulsion de stimulation VNS.
La Figure 6 illustre une première manière de réaliser l'invention, par adaptation d'un stimulateur cardiaque simple, double ou triple chambre pour la production d'un profil de neurostimulation non synchronisée, tel que celui de la Figure 3.
La Figure 7 illustre une deuxième manière de réaliser l'invention, par adaptation d'un stimulateur cardiaque double chambre pour la production d'un profil de neurostimulation synchrone, tel que celui de la Figure 2.
La Figure 8 illustre une troisième manière de réaliser l'invention, par adaptation d'un défibrillateur cardiaque pour la production d'un profil de neurostimulation synchrone, tel que celui de la Figure 2.
La Figure 9 illustre une quatrième manière de réaliser l'invention, par adaptation d'un resynchronisateur (dispositif CRT) pour la production d'un profil de neurostimulation synchrone, tel que celui de la Figure 2.

On va maintenant décrire un exemple de réalisation du dispositif de l'invention.

En ce qui concerne ses aspects logiciels, l'invention peut être mise en oeuvre par une programmation ou reprogrammation appropriée du logiciel de commande d'un dispositif connu de gestion du rythme cardiaque, par exemple un dispositif de type stimulateur cardiaque, défibrillateur et/ou resynchronisateur, comprenant des moyens d'acquisition d'un signal fourni par des sondes endocavitaires et/ou un ou plusieurs capteurs implantés.

L'invention peut notamment être appliquée aux dispositifs implantables tels que ceux des familles *Reply* (stimulateurs cardiaques) et *Paradym* (défibrillateurs et resynchronisateurs) produits et commercialisés par Sorin CRM, Clamart, France.

Il s'agit de dispositifs à microprocesseur programmable comportant des circuits pour recevoir, mettre en forme et traiter des signaux électriques recueillis par des électrodes implantées, et délivrer des impulsions de stimulation à ces électrodes. Il est possible d'y transmettre par télémétrie des logiciels qui seront conservés en mémoire et exécutés pour mettre en oeuvre les fonctions de l'invention qui seront décrites ci-dessous. L'adaptation de ces appareils à la mise en oeuvre des fonctions de l'invention est à la portée de l'homme du métier, et elle ne sera pas décrite en détail.

L'invention est mise en oeuvre par des moyens principalement logiciels, au moyen d'algorithmes appropriés exécutés par un microcontrôleur ou un processeur numérique de signal. Pour la clarté de l'exposé, les divers traitements appliqués seront décomposés et schématisés par un certain nombre de blocs fonctionnels distincts présentés sous forme de circuits interconnectés, mais cette représentation n'a toutefois qu'un caractère illustratif, ces circuits comprenant des éléments communs et correspondant en pratique à une pluralité de fonctions globalement exécutées par un même logiciel.

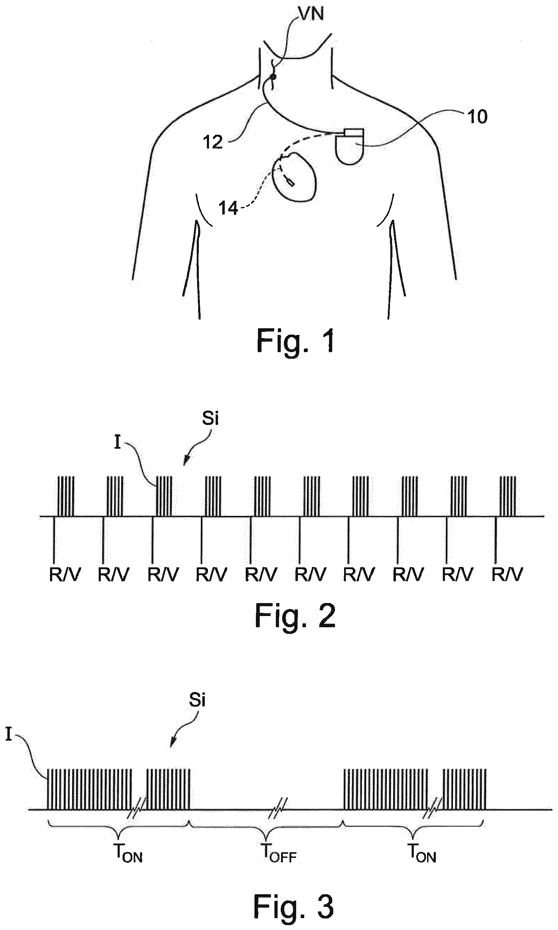

Sur la Figure 1, la référence 10 désigne le boitier d'un générateur implantable de stimulation du nerf vague (stimulation VNS). Cette stimulation est délivrée par une sonde 12 portant à sa partie distale une électrode implantée sur le nerf vague VN et susceptible de stimuler celui-ci par application de salves d'impulsions VNS produites par le générateur 10. Si le profil de stimulation VNS prévoit la délivrance d'impulsions en synchronisme avec le rythme cardiaque, le générateur 10 dispose également d'une sonde cardiaque 14 pourvue à son extrémité distale d'une électrode de recueil de l'activité électrique du myocarde. Cette sonde recueillera des signaux d'électrogramme endocavitaire EGM qui permettront de piloter le générateur 10 afin que celui-ci délivre au nerf vague des impulsions de stimulation VNS au même rythme que les battements cardiaques.

Sur la Figure 2, on a illustré un premier profil de neurostimulation, dans lequel le générateur délivre des salves Sᵢ d'impulsions VNS en synchronisme avec le rythme cardiaque, ce dernier étant typiquement matérialisé et suivi par des marqueurs correspondant aux instants de survenue de l'onde R, représentative du pic de dépolarisation spontanée des ventricules, ou des instants V d'application d'une impulsion de stimulation ventriculaire. D'autres techniques de détection du rythme peuvent être employées en variante ou en complément d'une détection du complexe QRS (marqueurs R ou V), par exemple des techniques basées sur la détection de l'onde P (détection auriculaire), voire de l'onde T (repolarisation ventriculaire).

Les salves Sᵢ d'impulsions VNS sont appliquées avec ou sans délai après l'onde R/V. Chaque salve Sᵢ est constituée d'une pluralité d'impulsions individuelles I en nombre réduit, par exemple deux à quatre impulsions successives. Les impulsions ont même amplitude et même largeur, de sorte que les impulsions délivrent individuellement la même énergie de stimulation VNS. L'intervalle entre deux impulsions I successives d'une même salve est de préférence un intervalle constant. Ce type de profil de neurostimulation est bien adapté au traitement des pathologies cardiaques, notamment pour les patients en risque d'insuffisance cardiaque.

La Figure 3 illustre un autre type de profil de neurostimulation, non synchronisé sur le rythme cardiaque, convenant par exemple au traitement de pathologies qui ne sont pas directement liées au fonctionnement du coeur, telles que l'épilepsie ou l'obésité. Ce profil consiste à délivrer des salves prolongées d'impulsions VNS pendant des périodes dites "d'activité" Tₒₙ, entrecoupées de périodes "d'inactivité" T_{off}. La fréquence de récurrence des impulsions I produites par le générateur VNS est typiquement comprise entre 8 et 50 Hz, avec des salves Sᵢ produites pendant des périodes d'activité Tₒₙ = 30 secondes alternant avec des périodes d'inactivité T_{off} = 5 minutes. Des pathologies cardiaques peuvent également êtres traités avec un tel profil de stimulation alternant périodes d'activité et d'inactivité, dès lors que les impulsions délivrées au cours des périodes d'activité le sont en synchronisme avec le rythme cardiaque.

L'idée de base de l'invention, comme exposée en introduction, consiste à utiliser pour produire ces salves d'impulsions VNS non pas un générateur VNS (dispositif médical dédié), mais un générateur de stimulation cardiaque (stimulateur, défibrillateur et/ou resynchronisateur), qu'il s'agisse :
- d'un dispositif destiné à une première implantation : il est alors possible de rationaliser la fabrication de ce dispositif, en le réalisant à partir des mêmes éléments matériels, la transformation de la fonction de stimulation cardiaque en stimulation VNS ne résultant que d'une modification logicielle ;
- ou même d'un dispositif cardiaque déjà implanté chez un patient : l'intervention se limitera alors à la pose et au raccordement d'une sonde VNS et à la reprogrammation logicielle du générateur, sans échange ni même explantation de celui-ci.

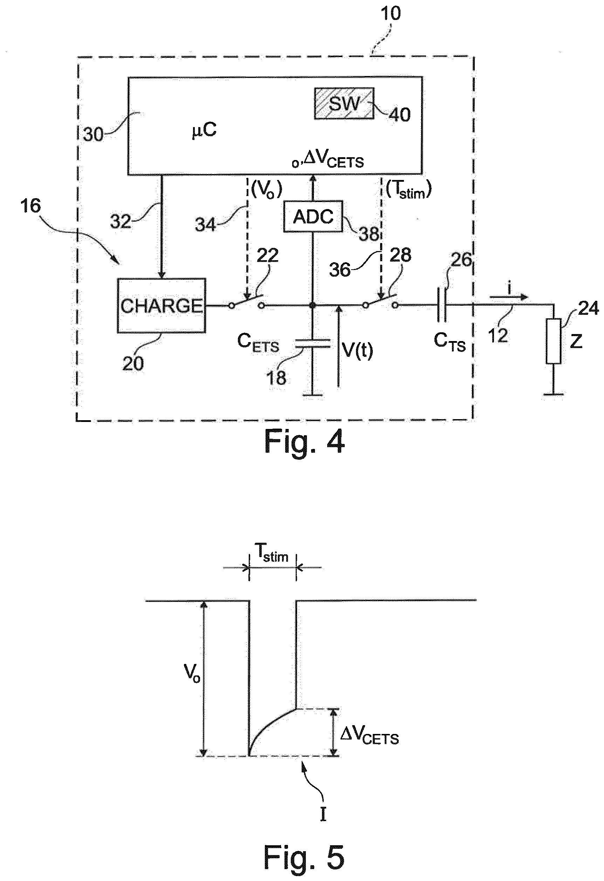

La Figure 4 est une représentation schématique par blocs des circuits de stimulation et de pilotage de l'un des canaux du générateur 10 utilisé pour la mise en oeuvre de l'invention.

Le circuit de stimulation 16 est un circuit conventionnel de stimulateur cardiaque, avec un condensateur réservoir 18 de capacité CETS chargé au préalable à une tension de charge V₀ par un circuit de charge 20, après fermeture du commutateur 22. Une fois la tension de charge préalable V₀ atteinte, ce condensateur 18 est prêt à être déchargé dans les tissus du patient, l'interface électrode/tissus et les tissus proprement dits étant électriquement modélisés en 24 par une impédance de valeur Z à l'extrémité de la sonde 12 reliant le générateur 10 au site de stimulation. La décharge du condensateur 18 intervient via un condensateur de liaison 26 de capacité CTS et un interrupteur 28 contrôlé de manière à décharger le condensateur 18 pendant une durée Tₛₜᵢₘ correspondant à la durée programmée de l'impulsion de stimulation.

Cette impulsion de stimulation est illustrée sur la Figure 5 avec les différents paramètres qui la caractérisent, à savoir la tension de charge préalable V₀, la durée de l'impulsion Tₛₜᵢₘ et la chute de tension ΔV_{CETS} observée aux bornes du condensateur 18 entre l'instant de fermeture et l'instant d'ouverture de l'interrupteur commandé 28.

Le contrôle des différents composants matériels que l'on vient de décrire est assuré par un circuit de pilotage 30 qui détermine la tension préalable de charge V₀ du circuit de charge 20 (flèche 32) ainsi que le séquencement des interrupteurs contrôlés de charge 22 et de décharge 28 (flèches 34 et 36). Le circuit de pilotage 30 reçoit par ailleurs en entrée un signal de mesure de la tension instantanée V(t) aux bornes du condensateur 18 via un convertisseur analogique/numérique 38.

En particulier, le temps de fermeture de l'interrupteur de charge 22 détermine la valeur V₀ de la tension de charge préalable amenée et maintenue aux bornes du condensateur 18 avant que celui-ci ne soit déchargé. En d'autres termes, après la délivrance d'une impulsion, le circuit de pilotage 30 ferme l'interrupteur 22 pour recharger le condensateur 18 et, une fois que le circuit de mesure 38 indique que la tension aux bornes de ce condensateur est V(t) = V₀, il ouvre l'interrupteur 22 afin de maintenir à cette valeur de consigne V₀ la tension aux bornes du condensateur 18 jusqu'au moment de la délivrance de l'impulsion. Le circuit de mesure 38 permet ensuite au circuit de pilotage 30 de déterminer le paramètre de chute de tension ΔV_{CETS} entre le début et la fin de l'impulsion de stimulation.

Les circuits que l'on vient de décrire sont en eux-mêmes connus. On pourra par exemple se référer au EP 1 216 723 A1 (ELA Medical), qui explique en outre la manière d'évaluer l'impédance Z à partir de mesures opérées sur le profil de l'impulsion délivrée aux tissus via la sonde.

Selon l'état de la technique, cette procédure d'évaluation de l'impédance n'est opérée que de temps à autre, pour déterminer l'évolution au cours du temps des caractéristiques électriques de l'interface sonde/myocarde. Ces caractéristiques peuvent en effet évoluer pour diverses raisons, en particulier la modification de l'environnement de la tête de sonde (formation de tissus réactionnels de contact) et l'altération du matériau conducteur formant l'électrode de la sonde.

Il s'agit ainsi de mesurer l'impédance de la sonde pour voir si celle-ci reste dans des limites acceptables et réajuster éventuellement les paramètres électriques de délivrance de l'impulsion de stimulation, notamment pour éviter une consommation excessive d'énergie qui pourrait nuire à la durée de vie du générateur.

Dans le cas de la présente invention, le circuit d'évaluation de l'impédance est utilisé à une autre fin : il s'agit de déterminer à partir des paramètres mesurés V₀ et ΔV_{CETS}, et connaissant Tₛₜᵢₘ, la charge (quantité de courant) délivrée par l'impulsion I, et de réajuster, d'une impulsion à la suivante et d'une salve à la suivante, la tension de charge préalable V₀ du condensateur - qui devient donc un paramètre modifiable à chaque impulsion - afin que la charge délivrée aux tissus soit une charge quasiment constante c'est-à-dire que, pour une même durée d'impulsion Tₛₜᵢₘ, le courant moyen délivré est un courant quasi constant.

On est ainsi sûr que chaque impulsion de stimulation délivrée au nerf vague produira le même effet physiologique, malgré les variations de l'interface entre le matériau conducteur de l'électrode et le nerf vague, dont l'impédance est susceptible de variations significatives sur des périodes relativement courtes (l'interface nerveuse étant beaucoup plus instable que l'interface cardiaque).

Au début du processus, le circuit de pilotage 30 évalue la charge délivrée par l'impulsion et compare celle-ci à une valeur cible, plus précisément en comparant la chute de tension ΔV_{CETS} mesurée à une consigne définie par une chute de tension ΔV₀ correspondant à la valeur cible. La tension préalable V₀ de charge du condensateur est alors réajustée pour les impulsions suivantes en fonction de l'écart mesure/consigne ainsi déterminé, dans un sens allant dans la réduction de cet écart. Une fois la valeur cible atteinte, on considère que la charge délivrée à chaque impulsion au nerf vague correspond à la valeur souhaitée, l'asservissement se réduisant alors à un simple *tracking* (poursuite) de stabilisation autour de la valeur cible.

Comme on le comprendra, la mise en oeuvre de ce processus d'ajustement est opérée au sein du circuit de pilotage 30, qui permet l'ajustement cycle-à-cycle de la tension de charge préalable V₀ et le séquencement (durée, fréquence de répétition) des salves d'impulsions de stimulation.

Un tel circuit est généralement architecturé autour d'un microcontrôleur, auquel il suffira d'implanter un module logiciel 40 dédié à la stimulation VNS, sans modification d'aucun des éléments matériels contrôlés par le circuit de pilotage 30 : condensateur 18, circuit de charge 20, commutateur 22, 28 de charge/décharge, circuit 38 de mesure de la tension aux bornes du condensateur.

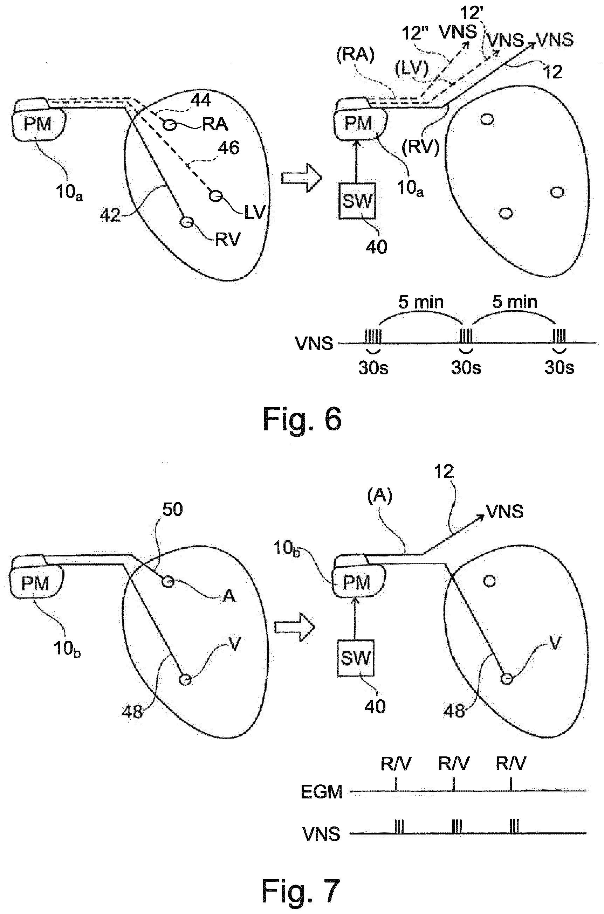

Les Figures 6 à 9 illustrent diverses manières de mettre en oeuvre l'invention à partir de dispositifs cardiaques implantés ou implantables préexistants.

Sur ces figures, la partie gauche illustre le dispositif cardiaque (stimulateur, défibrillateur ou resynchronisateur) dans sa configuration conventionnelle, c'est-à-dire où il est utilisé en combinaison avec des sondes aboutissant à des sites de stimulation situés sur le myocarde, tandis que la partie de droite illustre ce même dispositif dans son utilisation selon les enseignements de l'invention, c'est-à-dire en tant que dispositif de stimulation VNS, relié à une sonde aboutissant à un site de stimulation situé sur le nerf vague.

Les sondes VNS sont en elles-mêmes connues, par exemple celles produites et commercialisées par la société Neurotech SA, Louvain-la-Neuve, Belgique. Elles peuvent être adaptées à l'invention de façon simple en modifiant le connecteur de sonde, avec remplacement du connecteur propriétaire (destiné au couplage à un neurostimulateur implantable dédié) par un connecteur permettant un branchement sur un stimulateur cardiaque, typiquement un connecteur de type normalisé IS-1.

L'autre modification à opérer est le chargement dans le microcontrôleur du générateur cardiaque d'un module logiciel 40 permettant de réaliser les fonctions décrites plus haut, notamment le séquencement des impulsions sous forme de salves contrôlées et, pour chaque impulsion, le contrôle de celle-ci pour qu'elle assure la délivrance au nerf d'une charge électrique quasiment constante.

La Figure 6 illustre une première configuration, où le dispositif de stimulation VNS est réalisé à partir d'un stimulateur cardiaque de type simple, double ou triple chambre, par exemple un stimulateur de la famille *Reply* de Sorin CRM.

Dans sa configuration conventionnelle (stimulation cardiaque), le générateur 10a comprend un, deux ou trois canaux de détection/stimulation avec une, deux ou trois bornes respectives, reliées à un site ventriculaire droit RV par une sonde 42 et le cas échéant à un site auriculaire droit RA par une sonde 44 et à un site ventriculaire gauche LV par une sonde 46.

Dans la configuration selon l'invention (stimulation VNS), le canal RV du générateur 10a est relié à une sonde VNS 12 et, le cas échéant, les canaux LV et RA à d'autres sondes VNS 12' et 12". En particulier, si l'on dispose de deux voies de stimulation VNS, il peut être avantageux de délivrer des signaux en opposition de phase sur le nerf vague.

Cette configuration de stimulation VNS est en particulier appropriée à la délivrance de trains d'impulsions non synchronisés tels que ceux qui ont été illustrés et décrits sur la Figure 3, c'est-à-dire des salves d'impulsions typiquement de fréquence comprise entre 8 et 50 Hz par exemple pendant des périodes Tₒₙ de 30 secondes entrecoupées de périodes T_{off} de 5 minutes. Un tel profil de neurostimulation non synchronisée sur le rythme cardiaque convient en particulier au traitement de pathologies telles que l'épilepsie ou l'obésité.

La Figure 7 illustre une deuxième configuration, où le dispositif de stimulation VNS est réalisé à partir d'un stimulateur cardiaque double chambre, par exemple un stimulateur de type *Reply* de Sorin CRM.

Dans sa configuration conventionnelle (stimulation cardiaque), le générateur 10b comprend une voie ventriculaire reliée à un site V par une sonde 48 et une voie auriculaire reliée à un site auriculaire par une sonde 50.

La modification selon l'invention (stimulation VNS) consiste, en conservant la voie ventriculaire et la sonde correspondante 48, à remplacer la sonde auriculaire 50 par une sonde VNS 12. En d'autres termes, la voie auriculaire est transformée en voie VNS, mais sans modification de la voie ventriculaire. En particulier, il est possible de recueillir au moyen de cette voie ventriculaire une information de rythme cardiaque (marqueur R/V), pour délivrer des impulsions selon un profil de neurostimulation synchrone tel que celui illustré à la Figure 2, typiquement avec délivrance de 2 à 4 impulsions sur le nerf vague à chaque cycle cardiaque détecté ou stimulé, la détection/stimulation étant assurée par la voie ventriculaire du stimulateur, qui n'a pas été modifiée. Un tel profil de neurostimulation synchronisée sur le rythme cardiaque convient en particulier au traitement de pathologies cardiaques telles que l'insuffisance cardiaque.

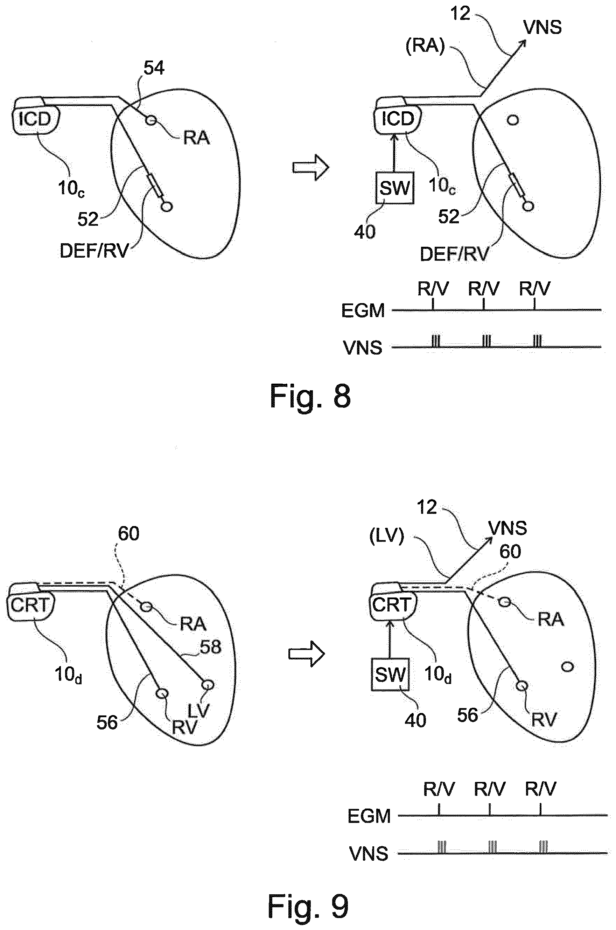

La Figure 8 illustre une troisième configuration, où le dispositif de stimulation VNS est réalisé à partir d'un défibrillateur/cardioverteur implantable, par exemple le modèle *Paradym DR* ou *Paradym VR* de Sorin CRM.

Dans sa configuration conventionnelle (stimulation cardiaque), le générateur 10c est relié à une sonde 52 comprenant un bobinage de défibrillation DEF et une ou plusieurs électrodes de détection/stimulation ventriculaire RV, ainsi qu'à une sonde 54 de détection/stimulation auriculaire RA.

La modification selon l'invention (stimulation VNS) consiste, comme dans le cas de la Figure 7, à remplacer la sonde auriculaire 54 par une sonde VNS 12 tout en conservant la voie ventriculaire DEF/RV et la sonde correspondante 52.

Les fonctionnalités de détection/stimulation sont ainsi conservées (les mêmes que dans le mode de réalisation précédent) ainsi que celles de délivrance d'un choc de défibrillation, tout en permettant au patient de bénéficier de surcroit d'une stimulation VNS avec un profil de neurostimulation synchronisée sur le rythme cardiaque.

La Figure 9 illustre une quatrième configuration possible, où le dispositif est un resynchronisateur, par exemple du type *Paradym CRT* de Sorin CRM, c'est-à-dire un dispositif de stimulation biventriculaire comprenant un générateur 10d relié par une sonde 56 à un site ventriculaire droit RV et par une sonde 58 à un site ventriculaire gauche LV. Le générateur peut également être relié à une sonde 60 de détection/stimulation auriculaire RA, présente sur une grande majorité des dispositifs CRT. On notera par ailleurs que le dispositif CRT peut être aussi bien un dispositif de stimulation pure (CRT-P) que de stimulation/défibrillation (CRT-D), la sonde ventriculaire portant dans ce dernier cas un bobinage de défibrillation comme dans le cas de la Figure 8.

La modification selon l'invention (stimulation VNS) consiste, tout en conservant la sonde 56 de détection ventriculaire droite, à relier la voie ventriculaire gauche à une sonde de stimulation VNS 12 pour assurer une stimulation VNS avec un profil de neurostimulation synchronisée sur le rythme cardiaque. Si le dispositif comporte une sonde auriculaire et/ou un bobinage de défibrillation, ces éléments sont conservés tels quels.

Cette quatrième configuration s'applique avantageusement à des patients qui, après avoir été implantés d'un dispositif CRT, montrent finalement qu'ils ne répondent pas à la thérapie CRT - ce qui est habituellement le cas d'environ 30 % de ces patients. Le dispositif implanté pourra dans ce cas être modifié pour faire bénéficier le patient d'une stimulation VNS, en remplacement ou en complément de la stimulation ventriculaire (qui demeure possible par la sonde 56 aboutissant au site ventriculaire droit RV).

L'intervention se limitera alors, en laissant le générateur 10d en place, à déconnecter de celui-ci la sonde ventriculaire gauche 58 (qui peut être laissée en place ou bien explantée) et, à la place, à implanter une sonde spécifique de neurostimulation munie d'un connecteur approprié (connecteur IS-1 compatible avec un générateur de dispositif cardiaque) et enfin à connecter cette sonde à la voie LV du générateur. Ce dernier est bien entendu reprogrammé pour y charger le module logiciel 40 spécifique, dédié à la neurostimulation.

## Revendications

1. Dispositif médical actif, comprenant :
- un générateur cardiaque implantable (10) de stimulation, défibrillation et/ou resynchronisation du myocarde, comportant :
• au moins deux bornes de détection/stimulation, avec une première et une seconde borne ;
• un circuit de stimulation (16), apte à générer des impulsions électriques de stimulation ;
• un circuit de pilotage (30), apte à contrôler la délivrance au moins sur la première borne des impulsions générées par le circuit de stimulation et le séquencement de ces impulsions ; et
• un circuit de recueil d'un signal EGM d'électrogramme endocavitaire et de détection d'une onde de dépolarisation cardiaque spontanée ou stimulée sur chaque cycle cardiaque;
dans lequel :
- le dispositif comprend en outre au moins une sonde (12, 12', 12") de stimulation du nerf vague, VNS, apte à être implantée sur le nerf vague (VN) ou à proximité de celui-ci ;
- la sonde VNS (12, 12', 12") est pourvue de moyens de connexion adaptés à un générateur cardiaque ;
- la sonde VNS (12, 12', 12") est reliée à la première borne du générateur cardiaque de manière à recevoir les impulsions produites sur cette borne par le générateur cardiaque afin d'appliquer ces impulsions sur le nerf vague ;
- le circuit de pilotage (30, 40) du générateur cardiaque est apte à contrôler le circuit de stimulation de manière à produire des salves (Sᵢ) d'impulsions (I), ces salves étant des salves VNS générées continûment en succession pendant des périodes d'activité (T_{ON}) séparées par des périodes intercalaires d'inactivité (T_{OFF}), et/ou générées en synchronisme sur détection d'une onde (R/V) de dépolarisation cardiaque,
**caractérisé en ce que** :
- le circuit de pilotage (30, 40) est en outre apte à mesurer l'impédance de la sonde VNS et, en réponse, à contrôler dynamiquement la tension d'impulsion (V₀) générée par le circuit de stimulation (16) de manière à ajuster à une valeur constante, d'une impulsion (I) à la suivante et d'une salve (Sᵢ) à la suivante, la charge délivrée par chaque impulsion de stimulation (I).

2. Le dispositif de la revendication 1, dans lequel lesdits moyens de connexion adaptés à un générateur cardiaque comprennent un connecteur de type IS-1 monté sur la sonde VNS.

3. Le dispositif de la revendication 1, dans lequel :
- le circuit de stimulation (16) comprend un condensateur (18) et des moyens (20, 22, 28) de charge et de décharge du condensateur sélectivement contrôlés (32, 34, 36) par le circuit de pilotage (30) ; et
- le circuit de pilotage (30, 40) comprend :
• des moyens (38) de mesure i) de la tension de charge préalable (V₀) du condensateur et ii) de la chute de la tension (ΔV_{CETS}) aux bornes de ce condensateur pendant la durée de la décharge ;
• des moyens (40) de calcul, à partir desdites mesures de tension de charge préalable (V₀) et de chute de tension (ΔV_{CETS}), de la charge électrique effectivement délivrée par l'impulsion de stimulation (I) produite par la décharge du condensateur ;
• des moyens (40) de calcul de l'écart entre la charge électrique ainsi calculée et une valeur de consigne correspondant à ladite valeur constante de charge électrique délivrée à chaque impulsion ; et
• des moyens (40) d'ajustement de la tension de charge préalable (V₀) du condensateur en fonction de l'écart ainsi calculé, dans un sens allant dans la réduction de cet écart.

4. Logiciel de conversion d'un générateur cardiaque implantable de stimulation, défibrillation et/ou resynchronisation du myocarde en un générateur VNS de stimulation du nerf vague, **caractérisé en ce qu'**il comprend des instructions aptes, lorsqu'elles sont exécutées par un dispositif selon la revendication 1, à exécuter des étapes de :
- configuration du circuit de pilotage (30, 40) du générateur cardiaque pour le rendre apte à contrôler le circuit de stimulation de manière à produire des salves d'impulsions, ces salves étant des salves (Sᵢ) d'impulsions (I), ces salves étant des salves VNS générées continûment en succession pendant des périodes d'activité (T_{ON}) séparées par des périodes intercalaires d'inactivité (T_{OFF}), et/ou générées en synchronisme sur détection d'une onde (R/V) de dépolarisation cardiaque ; et
- configuration du circuit de pilotage (30, 40) pour le rendre apte à mesurer l'impédance de la sonde VNS et, en réponse, à contrôler dynamiquement la tension d'impulsion (V₀) générée par le circuit de stimulation (16) de manière à ajuster à une valeur constante, d'une impulsion (I) à la suivante et d'une salve (Sᵢ) à la suivante, la charge délivrée par chaque impulsion de stimulation (I).

5. Le dispositif de la revendication 1 ou le logiciel de la revendication 4, dans lesquels :
- le générateur cardiaque (10) est un générateur (10a) de stimulateur mono-, double- ou triple chambre comprenant respectivement une, deux ou trois bornes de détection/stimulation ;
- chacune des bornes des stimulation est reliée à une sonde respective (12, 12', 12") de stimulation du nerf vague ; et
- le circuit de pilotage (30, 40) du générateur cardiaque est apte à contrôler le circuit de stimulation de manière à produire les salves VNS (Sᵢ) continûment en succession pendant des périodes d'activité (T_{ON}) séparées par des périodes intercalaires d'inactivité (T_{OFF}), non synchronisées sur la détection d'une onde de dépolarisation cardiaque.

6. Le dispositif de la revendication 1 ou le logiciel de la revendication 4, dans lesquels :
le générateur cardiaque (10) est un générateur (10b) de stimulateur double chambre où la première borne est une borne auriculaire et la seconde borne est une borne ventriculaire ;
le dispositif comprend en outre une sonde de détection ventriculaire (48), apte à être implantée dans une cavité ventriculaire droite du myocarde ;
la borne auriculaire est une borne de stimulation reliée à une sonde (12) de stimulation du nerf vague ;
la borne ventriculaire est une borne de détection/stimulation reliée à la sonde de détection ventriculaire (48) ;
le circuit de recueil d'un signal EGM et de détection d'une onde de dépolarisation cardiaque est couplé à la borne ventriculaire ; et
le circuit de pilotage (30, 40) du générateur cardiaque est apte à contrôler le circuit de stimulation de manière à produire les salves VNS (Sᵢ) en synchronisme sur les ondes de dépolarisation cardiaque (R/V) recueillies par la sonde de détection ventriculaire.

7. Le dispositif de la revendication 1 ou le logiciel de la revendication 4, dans lesquels :
- le générateur cardiaque (10) est un générateur (10c) de défibrillateur où la première borne est une borne auriculaire et la seconde borne est une borne ventriculaire ;
- le dispositif comprend en outre une sonde ventriculaire (52) comprenant une électrode de choc, apte à être implantée dans une cavité ventriculaire droite du myocarde ;
- la borne auriculaire est une borne de stimulation reliée à une sonde (12) de stimulation du nerf vague ;
- la borne ventriculaire est une borne de détection/stimulation/défibrillation reliée à la sonde comprenant l'électrode de choc ;
- le circuit de recueil d'un signal EGM et de détection d'une onde de dépolarisation cardiaque est couplé à la borne ventriculaire ; et
- le circuit de pilotage (30, 40) du générateur cardiaque est apte à contrôler le circuit de stimulation de manière à produire les salves VNS (Sᵢ) en synchronisme sur les ondes de dépolarisation cardiaque (R/V) recueillies par la sonde ventriculaire (52).

8. Le dispositif de la revendication 1 ou le logiciel de la revendication 4, dans lesquels :
- le générateur cardiaque (10) est un générateur (10d) de resynchronisation où la première borne est une borne ventriculaire gauche et la seconde borne est une borne ventriculaire droite ;
- le dispositif comprend en outre une sonde de détection ventriculaire droite (56), apte à être implantée dans une cavité ventriculaire droite du myocarde ;
- la borne ventriculaire gauche est une borne de stimulation reliée à une sonde (12) de stimulation du nerf vague ;
- la borne ventriculaire droite est une borne de détection/stimulation reliée à la sonde de détection ventriculaire droite ;
- le circuit de recueil d'un signal EGM et de détection d'une onde de dépolarisation cardiaque est couplé à la borne ventriculaire droite ; et
- le circuit de pilotage (30, 40) du générateur cardiaque est apte à contrôler le circuit de stimulation de manière à produire les salves VNS (Si) en synchronisme sur les ondes de dépolarisation cardiaque (R/V) recueillies par la sonde de détection ventriculaire droite (56).

## Patentansprüche

1. Aktive medizinische Einrichtung, umfassend:
- einen implantierbaren Herzschrittmacher (10) zur Stimulation, Defibrillation und/oder Resynchronisation des Herzmuskels, umfassend:
• mindestens zwei Detektions-/Stimulationsanschlüsse, mit einem ersten und einem zweiten Anschluss;
• eine Stimulationsschaltung (16), die befähigt ist, elektrische Stimulationsimpulse zu erzeugen;
• eine Steuerschaltung (30), die befähigt ist, die Ausgabe von Impulsen, welche durch die Stimulationssschaltung erzeugt werden, und die Abfolge dieser Impulse mindestens an dem ersten Anschluss zu steuern; und
• eine Schaltung zum Erfassen eines EGM-Signals eines endokavitären Elektrogramms und zum Erkennen einer spontanen oder stimulierten kardialen Depolarisationswelle bei jedem Herzzyklus;
wobei:
- die Vorrichtung ferner mindestens eine Sonde (12, 12', 12") zur Vagusnervstimulation, VNS, umfasst, die zur Implantation am Vagusnerv (VN) oder in dessen Nähe befähigt ist;
- die VNS-Sonde (12, 12', 12") mit geeigneten Mitteln zum Anschluss an einen Herzschrittmacher ausgestattet ist;
- die VNS-Sonde (12, 12', 12") mit dem ersten Anschluss des Herzschrittmachers verbunden ist, sodass die an diesem Anschluss vom Herzschrittmacher erzeugten Impulse empfangen werden, um diese Impulse am Vagusnerv anzulegen;
- die Steuerschaltung (30, 40) des Herzschrittmachers befähigt ist, die Stimulationsschaltung so zu steuern, dass Bursts (Si) von Impulsen (I) erzeugt werden, wobei diese Bursts VNS-Bursts sind, die kontinuierlich nacheinander während Aktivitätszeiten (T_{ON}) erzeugt werden, welche durch dazwischen liegende Zeiten der Inaktivität (T_{OFF}) getrennt sind, und/oder die synchron zum Erkennen einer kardialen Depolarisationswelle (R/V) erzeugt werden,
**dadurch gekennzeichnet, dass**:
- die Steuerschaltung (30, 40) ferner befähigt ist, die Impedanz der VNS-Sonde zu messen und als Reaktion die von der Stimulationsschaltung (16) erzeugte Impulsspannung (Vₒ) dynamisch zu steuern, sodass die bei jedem Stimulationsimpuls (I) abgegebene Ladung von einem Impuls (I) zum nächsten und von einem Burst (Si) zum nächsten, auf einen konstanten Wert eingestellt wird.

2. Vorrichtung nach Anspruch 1, wobei die an einen Herzschrittmacher angepassten Anschlussmittel einen Steckverbinder vom Typ IS-1 beinhalten, der an die VNS-Sonde gekoppelt ist.

3. Vorrichtung nach Anspruch 1, wobei:
- die Stimulationsschaltung (16) einen Kondensator (18) und Mittel (20, 22, 28) zum Laden und Entladen des Kondensator umfasst, die von der Steuerschaltung (30) selektiv gesteuert (32, 34, 36) werden; und
- die Steuerschaltung (30, 40) umfasst:
• Mittel (38) zum Messen i) der Vorladespannung (Vₒ) des Kondensators und ii) des Spannungsabfalls (ΔV_{CETS}) an den Anschlüssen dieses Kondensators während der Entladungsdauer;
• Mittel (40) zum Berechnen, aus den Messungen der Vorladespannung (V₀) und des Spannungsabfalls (ΔV_{CETS}), der elektrischen Ladung, die tatsächlich von dem durch das Entladen des Kondensators erzeugten Stimulationsimpuls (I) abgegeben wird;
• Mittel (40) zum Berechnen der Differenz zwischen der so berechneten elektrischen Ladung und einem Sollwert, der dem konstanten Wert der elektrischen Ladung entspricht, die bei jedem Impuls abgegebenen wird; und
• Mittel (40) zum Einstellen der Vorladespannung (Vₒ) des Kondensators in Abhängigkeit von der so berechneten Differenz, in eine Richtung, die zur Reduzierung dieser Differenz führt.

4. Software zum Umwandeln eines implantierbaren Herzschrittmachers zur Stimulation, Defibrillation und/oder Resynchronisation des Herzmuskels in einen VNS-Generator zur Vagusnervstimulation, **dadurch gekennzeichnet, dass** die Software Anweisungen enthält, die, wenn sie von einer Vorrichtung nach Anspruch 1 ausgeführt werden, zum Ausführen von folgenden Schritten befähigt sind:
- Konfiguration der Steuerschaltung (30, 40) des Herzschrittmachers, um ihn zu befähigen, die Stimulationsschaltung so zu steuern, dass Impulsbursts erzeugt werden, wobei diese Bursts Impuls-(I)-Bursts (Si) sind, wobei diese Bursts VNS-Bursts sind, die kontinuierlich nacheinander während Aktivitätszeiten (T_{ON}) erzeugt werden, welche durch dazwischen liegende Zeiten der Inaktivität (T_{OFF}) getrennt sind, und/oder die synchron zum Erkennen einer kardialen Depolarisationswelle (R/V) erzeugt werden; und
- Konfiguration der Steuerschaltung (30, 40), um sie zu befähigen, die Impedanz der VNS-Sonde zu messen und als Reaktion die von der Stimulationsschaltung (16) erzeugte Impulsspannung (Vₒ) dynamisch zu steuern, sodass die bei jedem Stimulationsimpuls (I) abgegebene Ladung von einem Impuls (I) zum nächsten und von einem Burst (Si) zum nächsten auf einen konstanten Wert eingestellt wird.

5. Vorrichtung nach Anspruch 1 oder Software nach Anspruch 4, wobei:
- der Herzschrittmacher (10) ein Ein-, Zwei- oder Dreikammer-Stimulationsschrittmacher (10a) ist, der jeweils einen, zwei oder drei Detektions-/Stimulationsanschlüsse umfasst;
- jeder der Stimulatonsanschlüsse mit einer entsprechenden Vagusnerv-Stimulationssonde (12, 12', 12") verbunden ist; und
- die Steuerschaltung (30, 40) des Herzschrittmachers befähigt ist, die Stimulationsschaltung so zu steuern, dass VNS-Bursts (Si) kontinuierlich nacheinander während Aktivitätszeiten (T_{ON}) erzeugt werden, welche durch dazwischen liegende Zeiten der Inaktivität (T_{OFF}) getrennt sind, die nicht mit der Erkennung einer kardialen Depolarisationswelle synchronisiert sind.

6. Vorrichtung nach Anspruch 1 oder Software nach Anspruch 4, wobei:
der Herzschrittmacher (10) ein Zweikammer-Stimulationsschrittmacher (10b) ist, wobei der erste Anschluss ein atrialer Anschluss und der zweite Anschluss ein ventrikulärer Anschluss ist;
die Vorrichtung ferner eine ventrikuläre Detektionssonde (48) umfasst, die zur Implantation in eine rechte Ventrikelhöhle des Herzmuskels befähigt ist;
der atriale Anschluss ein Stimulationsanschluss ist, der mit einer Vagusnerv-Stimulationssonde (12) verbunden ist;
der ventrikuläre Anschluss ein Detektions-/Stimulationsanschluss ist, der mit der ventrikulären Detektionssonde (48) verbunden ist;
die Schaltung zum Erfassen eines EGM-Signals und zum Erkennen einer kardialen Depolarisationswelle mit dem ventrikulären Anschluss gekoppelt ist; und
die Steuerschaltung (30, 40) des Herzschrittmachers befähigt ist, die Stimulationsschaltung so zu steuern, dass die VNS-Bursts (Si) synchron zu den kardialen Depolarisationswellen (R/V) erzeugt werden, die von der ventrikulären Detektionssonde erfasst werden.

7. Vorrichtung nach Anspruch 1 oder Software nach Anspruch 4, wobei:
- der Herzschrittmacher (10) ein Defibrillationsschrittmacher (10c) ist, wobei der erste Anschluss ein atrialer Anschluss und der zweite Anschluss ein ventrikulärer Anschluss ist;
- die Vorrichtung ferner eine ventrikuläre Sonde (52) umfasst, welche eine Schockelektrode umfasst, die zur Implantation in eine rechte Ventrikelhöhle des Herzmuskels befähigt ist;
- der atriale Anschluss ein Stimulationsanschluss ist, der mit einer Vagusnerv-Stimulationssonde (12) verbunden ist;
- der ventrikuläre Anschluss ein Detektions-/Stimulations-/Defibrillationsanschluss ist, der mit der Sonde verbunden ist, die die Schockelektrode enthält;
- die Schaltung zum Erfassen eines EGM-Signals und zum Erkennen einer kardialen Depolarisationswelle mit dem ventrikulären Anschluss gekoppelt ist; und
- die Steuerschaltung (30, 40) des Herzschrittmachers befähigt ist, die Stimulationsschaltung so zu steuern, dass die VNS-Bursts (Si) synchron zu den kardialen Depolarisationswellen (R/V) erzeugt werden, die von der ventrikulären Sonde (52) erfasst werden.

8. Vorrichtung nach Anspruch 1 oder Software nach Anspruch 4, wobei:
- der Herzschrittmacher (10) ein Resynchronisationsschrittmacher (10d) ist, wobei der erste Anschluss ein linksventrikulärer Anschluss und der zweite Anschluss ein rechtsventrikulärer Anschluss ist;
- die Vorrichtung ferner eine rechtsventrikuläre Detektionssonde (56) umfasst, die zur Implantation in eine rechte Ventrikelhöhle des Herzmuskels befähigt ist;
- der linksventrikuläre Anschluss ein Stimulationsanschluss ist, der mit einer Vagusnerv-Stimulationssonde (12) verbunden ist;
- der rechtsventrikuläre Anschluss ein Detektions-/Stimulationsanschluss ist, der mit der rechtsventrikulären Detektionssonde verbunden ist;
- die Schaltung zum Erfassen eines EGM-Signals und zum Erkennen einer kardialen Depolarisationswelle mit dem rechtsventrikulären Anschluss gekoppelt ist; und
- die Steuerschaltung (30, 40) des Herzschrittmachers befähigt ist, die Stimulationsschaltung so zu steuern, dass die VNS-Bursts (Si) synchron zu den kardialen Depolarisationswellen (R/V) erzeugt werden, die von der rechtsventrikulären Sonde (56) erfasst werden.

## Claims

1. An active medical device comprising:
- an implantable cardiac generator (10) for myocardial stimulation, defibrillation and/or resynchronization, the generator comprising:
• at least two detection/stimulation terminals, with a first terminal and a second terminal;
• a stimulation circuit (16) suitable for generating electrical stimulation pulses;
• a control circuit (30) suitable for controlling delivery, at least at the first terminal, of the pulses generated by the stimulation circuit, and for controlling sequencing of said pulses; and
• a collection circuit for collecting an endocardial electrogram (EGM) signal and for detecting a spontaneous or stimulated cardiac depolarization wave at each cardiac cycle;
in which active medical device:
- the device further comprises at least one lead (12, 12', 12") for vagus nerve stimulation (VNS), which lead is suitable for being implanted on or in the vicinity of the vagus nerve (VN);
- the VNS lead (12, 12', 12") is provided with connection means adapted to a cardiac generator;
- the VNS lead (12, 12', 12") is connected to the first terminal of the cardiac generator in such a manner as to receive the pulses produced at said terminal by the cardiac generator in order to apply said pulses to the vagus nerve;
- the control circuit (30, 40) of the cardiac generator is suitable for controlling the stimulation circuit in such a manner as to produce bursts (Si) of pulses (I), said bursts being VNS bursts that are generated continuously in succession for periods of activity (T_{ON}) separated by interspersed periods of inactivity (T_{OFF}), and/or that are generated synchronously with detection of a cardiac depolarization wave (R/V);
said active medical device being **characterized in that**:
- the control circuit (30, 40) is also suitable for measuring the impedance of the VNS lead, and for responding by dynamically controlling the pulse voltage (V₀) generated by the stimulation circuit (16) in such a manner as to adjust the charge delivered by each stimulation pulse (I) to a value that is constant from one pulse (I) to the next, and from one burst (Si) to the next.

2. The device of claim 1, wherein said connection means adapted to a cardiac generator comprise a connector of the IS-1 type mounted on the VNS lead.

3. The device of claim 1, wherein:
- the stimulation circuit (16) comprises a capacitor (18) and means (20, 22, 28) for charging and discharging the capacitor that are controlled selectively (32, 34, 36) by the control circuit (30); and
- the control circuit (30, 40) comprises:
• means (38) for measuring i) the prior charge voltage (V₀) of the capacitor and ii) the drop in voltage (ΔV_{CETS}) across the terminals of said capacitor for the duration of the discharging;
• means (40) for acting on the basis of said measurements of the prior charge voltage (V₀) and of the voltage drop (ΔV_{CETS}) to compute the electric charge actually delivered by the stimulation pulse (I) produced by the discharging of the capacitor;
• means (40) for computing the difference between the electric charge computed in this way and a reference value corresponding to said constant value of electric charge delivered at each pulse; and
• means (40) for adjusting the prior charge voltage (V₀) of the capacitor as a function of the difference computed in this way, in such a manner as to reduce said difference.

4. Conversion software for converting an implantable cardiac generator for myocardial stimulation, defibrillation and/or resynchronization into a vagus nerve stimulation (VNS) generator, said conversion software being **characterized in that** it comprises instructions suitable, when they are executed by a device according to claim 1, for performing the steps of:
- configuring the control circuit (30, 40) of the cardiac generator for making it suitable for controlling the stimulation circuit in such a manner as to produce bursts (Si) of pulses (I), said bursts being VNS bursts that are generated continuously in succession for periods of activity (T_{ON}) separated by interspersed periods of inactivity (T_{OFF}), and/or that are generated synchronously with detection of a cardiac depolarization wave (R/V); and
- configuring the control circuit (30, 40) for making it suitable for measuring the impedance of the VNS lead, and for responding by dynamically controlling the pulse voltage (V₀) generated by the stimulation circuit (16) in such a manner as to adjust the charge delivered by each stimulation pulse (I) to a value that is constant from one pulse (I) to the next, and from one burst (Si) to the next.

5. The device of claim 1 or the software of claim 4, wherein:
- the cardiac generator (10) is a single-chamber, double-chamber, or triple-chamber generator (10a), respectively having one, two or three detection/stimulation terminals;
- each of the stimulation terminals is connected to a respective lead (12, 12', 12") for vagus nerve stimulation; and
- the control circuit (30, 40) of the cardiac generator is suitable for controlling the stimulation circuit in such a manner as to produce the VNS bursts (Si) continuously in succession for periods of activity (T_{ON}) separated by interspersed periods of inactivity (T_{OFF}), and not synchronously with detection of a cardiac depolarization wave (R/V).

6. The device of claim 1 or the software of claim 4, wherein:
the cardiac generator (10) is a double-chamber stimulator generator (10b) in which the first terminal is an atrial terminal and the second terminal is a ventricular terminal;
the device further comprises a ventricular detection lead (48), suitable for being implanted in a right ventricular cavity of the myocardium;
the atrial terminal is a stimulation terminal connected to a lead (12) for vagus nerve stimulation;
the ventricular terminal is a detection/stimulation terminal connected to the ventricular detection lead (48);
the collection circuit for collecting an EGM signal and for detecting a cardiac depolarization wave is coupled to the ventricular terminal; and
the control circuit (30, 40) of the cardiac generator is suitable for controlling the stimulation circuit in such a manner as to produce the VNS bursts (S) synchronously with the cardiac depolarization waves (R/V) collected by the ventricular detection lead.

7. The device of claim 1 or the software of claim 4, wherein:
- the cardiac generator (10) is a defibrillator generator (10c) in which the first terminal is an atrial terminal and the second terminal is a ventricular terminal;
- the device further comprises a ventricular lead (52) comprising a shock electrode, and suitable for being implanted in a right ventricular cavity of the myocardium;
- the atrial terminal is a stimulation terminal connected to a lead (12) for vagus nerve stimulation;
- the ventricular terminal is a detection/stimulation/defibrillation terminal connected to the lead comprising the shock electrode;
- the collection circuit for collecting an EGM signal and for detecting a cardiac depolarization wave is coupled to the ventricular terminal; and
- the control circuit (30, 40) of the cardiac generator is suitable for controlling the stimulation circuit in such a manner as to produce the VNS bursts (Si) synchronously with the cardiac depolarization waves (R/V) collected by the ventricular lead (52).

8. The device of claim 1 or the software of claim 4, wherein:
- the cardiac generator (10) is a resynchronization generator (10d) in which the first terminal is a left ventricular terminal and the second terminal is a right ventricular terminal;
- the device further comprises a right ventricular detection lead (56), suitable for being implanted in a right ventricular cavity of the myocardium;
- the left atrial terminal is a stimulation terminal connected to a lead (12) for vagus nerve stimulation;
- the right ventricular terminal is a detection/stimulation terminal connected to the right ventricular detection lead;
- the collection circuit for collecting an EGM signal and for detecting a cardiac depolarization wave is coupled to the right ventricular terminal; and
- the control circuit (30, 40) of the cardiac generator is suitable for controlling the stimulation circuit in such a manner as to produce the VNS bursts (Si) synchronously with the cardiac depolarization waves (R/V) collected by the right ventricular detection lead (56).
